# EUROPEAN PATENT SPECIFICATION

(11) **EP 4 611 647 B1**
(45) Date of publication and mention of the grant of the patent: **21.10.2026**
(21) Application number: 23825023.7
(22) Date of filing: 09.11.2023
(51) Int. Cl.: A61B 17/04

(54) **DEVICES AND SYSTEMS FOR DEPLOYING AN ANCHOR INTO TISSUE TO A PRESCRIBED DEPTH**
VORRICHTUNGEN UND SYSTEME ZUM EINSETZEN EINES ANKERS IN GEWEBE ZU EINER VORGESCHRIEBENEN TIEFE
DISPOSITIFS ET SYSTÈMES POUR DÉPLOYER UN DISPOSITIF D'ANCRAGE DANS UN TISSU À UNE PROFONDEUR PRÉDÉFINIE

(30) Priority: 10.11.2022 US 202263424373 P
(43) Date of publication of application: 10.09.2025
(73) Proprietor: Boston Scientific Scimed Inc., Maple Grove, Minnesota 55311 (US); Mayo Foundation for Medical Education and Research, Rochester, MN 55905 (US)
(72) Inventor: EGGERT, Joel T., Plymouth, Minnesota 55442 (US); SHUEY, Daniel, Pine City, Minnesota 55063 (US); GIESE, Troy Anthony, Blaine, Minnesota 55449 (US)
(74) Representative: Peterreins Schley
(86) International application number: PCT/US2023/079185
(87) International publication number: WO 2024/102880

(56) References cited:
- WO-A1-99/37216
- US-A1- 2001 027 321
- US-A1- 2007 112 383
- US-A1- 2022 008 201
- US-A1- 2022 142 778

## Description

### FIELD

The present disclosure relates generally to the field of implantable medical devices. In particular, the present disclosure relates to devices, systems, and methods for implanting a device, such as an anchor device, into tissue. More particularly, the present disclosure relates to medical devices, systems, and methods for delivering and deploying a device, such as an anchor, into tissue to a prescribed implant depth.

### BACKGROUND

Various medical procedures involve anchoring a device or system to tissue. For instance, heart repair procedures involving repair of heart valve leaflets include anchoring a chordae tendineae, extending from a heart leaflet at one end, to heart tissue. More particularly, an artificial chordae tendineae may be coupled from the heart valve leaflet to heart tissue, such as papillary muscle tissue or to the heart wall. A common technical challenge with such procedures is the potential for the anchor to dislodge, which may sometimes occur shortly after delivery. One suspected cause of such dislodging of an anchor is myocardial necrosis. There remains a need for securing devices with respect to an implant site in a manner which resists dislodging of the device from the implant site.

WO 99/37216 Al discloses bone anchor implantation devices and methods for their use. The bone anchor implantation devices and methods find particular application in maintaining or improving urinary continence by suspending or stabilizing the bladder neck. Protective sheaths for covering a bone anchor on a bone anchor implantation device are disclosed. The protective sheaths protect the bone anchor from contacting tissue during insertion and prevent contamination of the bone anchor.

### SUMMARY

This Summary is provided to introduce, in simplified form, a selection of concepts described in further detail below in the Detailed Description. This Summary is not intended to necessarily identify key features or essential features of the claimed subject matter, nor is it intended as an aid in determining the scope of the claimed subject matter. One of skill in the art will understand that each of the various aspects and features of the present disclosure may advantageously be used separately in some instances, or in combination with other aspects and features of the disclosure in other instances, whether or not described in this Summary. No limitation as to the scope of the claimed subject matter is intended by either the inclusion or non-inclusion of elements, components, or the like in this Summary.

The present invention relates to an anchor delivery and deployment device including an anchor garage configured to deliver an anchor therein; an atraumatic shield movable with respect to the anchor garage; and a biasing element comprising a plurality of elongated beams proximal to said atraumatic shield and biased into an elongated configuration along said anchor garage to bias said atraumatic shield toward the distal end of said anchor garage.

Optionally, the anchor delivery and deployment device includes an indicator of the relative positions of the anchor garage and the atraumatic shield. In some embodiments, the indicator is a visual indicator. In some embodiments, the indicator includes at least one radiopaque marker. In some embodiments, the indicator generates a signal indicating the relative positions of the anchor garage and the atraumatic shield. In some embodiments, the indicator indicates that the anchor delivery and deployment device has contacted tissue.

Additionally or alternatively, the anchor delivery and deployment device includes a limit stop positioned to limit relative movement between the atraumatic shield and the anchor garage. In some embodiments, the limit stop is positioned and configured to limit proximal retraction of the atraumatic shield with respect to the anchor garage.

In some embodiments, the distal end of the anchor garage is sharp to penetrate tissue, and the biasing element biases the atraumatic shield over the distal end of the anchor garage. In some embodiments, a limit stop is positioned to limit advancement of the sharp distal end of the anchor garage out of the atraumatic shield.

The biasing element comprises a plurality of elongated beams proximal to the atraumatic shield and biased into an elongated configuration along the anchor garage to bias the atraumatic shield toward the distal end of the anchor garage. In some embodiments, the beams are formed from the wall of the anchor garage.

In accordance with various principles of the present disclosure, an anchor delivery and deployment system includes an anchor; an anchor garage configured to deliver the anchor therein; an atraumatic shield movable with respect to the anchor garage; and a biasing element comprising a plurality of elongated beams proximal to said atraumatic shield and biased into an elongated configuration along said anchor garage to bias said atraumatic shield toward the distal end of said anchor garage.

Optionally, the anchor delivery and deployment system includes a tubular delivery element configured to deliver the anchor, the anchor garage, and the atraumatic shield transluminally through a patient's body to a target site.

Optionally, the anchor delivery and deployment system includes an indicator configured to indicate the relative positions of the anchor garage and the atraumatic shield.

Optionally, the anchor delivery and deployment system includes a limit stop positioned to limit relative movement of the atraumatic shield with respect to the anchor garage.

In accordance with various principles of the present disclosure, a method of delivering and deploying an anchor to a target site within a patient's body includes delivering an anchor within an anchor garage to a target site; contacting an atraumatic shield positioned with respect to the anchor garage with tissue at the target site to cause the atraumatic shield to retract proximally with respect to the anchor garage; deploying the anchor into the target site; proximally retracting the anchor garage from the target site to allow a biasing element to bias the atraumatic shield distally with respect to the anchor garage; and retracting the anchor garage and atraumatic shield from the target site.

The method optionally further includes causing an indicator positioned with respect to the atraumatic shield and the anchor garage to indicate the relative position of the atraumatic shield and the anchor garage.

The method optionally further includes distally advancing the anchor garage and the atraumatic shield against tissue to cause proximal retraction of the atraumatic shield with respect to the anchor garage until the atraumatic shield is inhibited from further proximal movement by encountering a limit stop.

The method optionally further includes allowing the biasing element to bias the atraumatic shield distally with respect to the anchor garage, and to return the biasing element to a configuration in which the anchor garage, the atraumatic shield, and the biasing element may be retracted into a delivery device for withdrawal from the patient.

These and other features and advantages of the present disclosure, will be readily apparent from the following detailed description, the scope of the claimed invention being set out in the appended claims.

### BRIEF DESCRIPTION OF THE DRAWINGS

Non-limiting embodiments of the present disclosure are described by way of example with reference to the accompanying drawings, which are schematic and not intended to be drawn to scale. The accompanying drawings are provided for purposes of illustration only, and the dimensions, positions, order, and relative sizes reflected in the figures in the drawings may vary. For example, devices may be enlarged so that detail is discernable, but is intended to be scaled down in relation to, *e.g.,* fit within a working channel of a delivery catheter or endoscope. In the figures, identical or nearly identical or equivalent elements are typically represented by the same reference characters, and similar elements are typically designated with similar reference numbers differing in increments of 100, with redundant description omitted. For purposes of clarity and simplicity, not every element is labeled in every figure, nor is every element of each embodiment shown where illustration is not necessary to allow those of ordinary skill in the art to understand the disclosure.

The detailed description will be better understood in conjunction with the accompanying drawings, wherein like reference characters represent like elements, as follows:
**FIG. 1** illustrates a perspective view of an example of an embodiment of an anchor delivery and deployment device and system formed in accordance with various principles of the present disclosure shown in a schematic representation of a heart for deployment in cardiac tissue.
**FIG. 2** illustrates a perspective view of an anchor delivery and deployment device and system as illustrated in **FIG. 1****,** with the anchor delivery and deployment device illustrated in a delivery configuration.
**FIG. 3** illustrates a perspective view similar to that of **FIG. 2****,** but in with the anchor delivery and deployment device in a deployment configuration.
**FIG. 4A** illustrates an elevational view of an anchor delivery and deployment device such as illustrated in **FIG. 1****,** delivering an anchor device into a tissue wall.
**FIG. 4B** illustrates an elevational view similar to that of **FIG. 4A****,** but after deployment of the anchor device and withdrawal of the anchor delivery and deployment device from the tissue wall.
**FIG. 5** illustrates a perspective view of another example of an embodiment of an anchor delivery and deployment device and system formed in accordance with various principles of the present disclosure.

### DETAILED DESCRIPTION

The following detailed description should be read with reference to the drawings, which depict illustrative embodiments. It is to be understood that the disclosure is not limited to the particular embodiments described, as such may vary. All apparatuses and systems and methods discussed herein are examples of apparatuses and/or systems and/or methods implemented in accordance with one or more principles of this disclosure. Each example of an embodiment is provided by way of explanation and is not the only way to implement these principles but are merely examples. Thus, references to elements or structures or features in the drawings must be appreciated as references to examples of embodiments of the disclosure, and should not be understood as limiting the disclosure to the specific elements, structures, or features illustrated. Other examples of manners of implementing the disclosed principles will occur to a person of ordinary skill in the art upon reading this disclosure. In fact, it will be apparent to those skilled in the art that various modifications and variations can be made in the present disclosure without departing from the scope of the present subject matter. For instance, features illustrated or described as part of one embodiment can be used with another embodiment to yield a still further embodiment. Thus, it is intended that the present subject matter covers such modifications and variations, the invention being defined by the claims.

It will be appreciated that the present disclosure is set forth in various levels of detail in this application. In certain instances, details that are not necessary for one of ordinary skill in the art to understand the disclosure, or that render other details difficult to perceive may have been omitted. The terminology used herein is for the purpose of describing particular embodiments only, and is not intended to be limiting beyond the scope of the appended claims. Unless defined otherwise, technical terms used herein are to be understood as commonly understood by one of ordinary skill in the art to which the disclosure belongs. All of the devices and/or methods disclosed and claimed herein can be made and executed without undue experimentation in light of the present disclosure.

As used herein, "proximal" refers to the direction or location closest to the user (medical professional or practitioner or clinician or technician or operator or physician, etc., such terms being used interchangeably herein without intent to limit, and including automated controller systems or otherwise), etc., such as when using a device (e.g., introducing the device into a patient, or during implantation, positioning, or delivery), and/or closest to a delivery device, and "distal" refers to the direction or location furthest from the user, such as when using the device (e.g., introducing the device into a patient, or during implantation, positioning, or delivery), and/or closest to a delivery device. "Longitudinal" means extending along the longer or larger dimension of an element. A "longitudinal axis" extends along the longitudinal extent of an element, though is not necessarily straight and does not necessarily maintain a fixed configuration if the element flexes or bends, and "axial" generally refers to along the longitudinal axis. However, it will be appreciated that reference to axial or longitudinal movement with respect to the above-described systems or elements thereof need not be strictly limited to axial and/or longitudinal movements along a longitudinal axis or central axis of the referenced elements. "Central" means at least generally bisecting a center point and/or generally equidistant from a periphery or boundary, and a "central axis" means, with respect to an opening, a line that at least generally bisects a center point of the opening, extending longitudinally along the length of the opening when the opening comprises, for example, a tubular element, a channel, a cavity, or a bore. As used herein, a "lumen" or "channel" or "bore" or "passage" is not limited to a circular cross-section. As used herein, a "free end" of an element is a terminal end at which such element does not extend beyond. It will be appreciated that terms such as at or on or adjacent or along an end may be used interchangeably herein without intent to limit unless otherwise stated, and are intended to indicate a general relative spatial relation rather than a precisely limited location. Finally, reference to "at" a location or site is intended to include at and/or about the vicinity of (e.g., along, adjacent, etc.) such location or site.

In accordance with various principles of the present disclosure, an implantable device, such as an anchor device, is deployed sufficiently deep into tissue to be firmly anchored thereto and to resist dislodging therefrom. It will be appreciated that the term anchor is used for the sake of convenience and may be used interchangeably herein with terms such as anchor component, anchor device, anchor element, anchor mechanism, anchoring component, anchoring device, anchoring element, anchoring mechanism, and the like, such terms being known in the art to represent structures configured to hold another object in place. For the sake of convenience and without intent to limit, reference is made herein simply to an anchor. It will be appreciated that references herein to anchors, including in the appended claims, are for the sake of convenience, and may encompass other medical devices to be implanted, unless clearly indicated otherwise. It will further be appreciated that terms such as implant (and other grammatical forms thereof) may be used interchangeably herein with terms (and grammatical forms thereof) such as affix, anchor, attach, associate, couple, engage, embed, hold, retain, purchase, secure, etc., without intent to limit.

In order to control the depth to which the anchor is deployed (e.g., to assure sufficient purchase with tissue, and optionally to limit excess depth of insertion such as not to pierce completely through a tissue wall into which the device is anchored), an anchor delivery and deployment device is configured to facilitate deployment of the anchor to a prescribed depth. A delivery device may be configured in accordance with various principles of the present disclosure to pierce tissue to a prescribed depth prior to deploying the anchor. The device may be further configured to indicate a prescribed depth of insertion has been achieved so that the anchor may then be deployed. For instance, it may be desirable to insert an anchor deep into tissue, where necrosis is less likely to occur. Moreover, fixation features on the anchor can be optimized for holding force independent of insertion depth. For instance, the width, thickness, number, spread, etc., of talons of the anchor can be adjusted to achieve the desired holding force with respect to tissue.

More particularly, in accordance with various principles of the present disclosure, an anchor delivery and deployment device includes an anchor garage with an internal lumen in which an anchor is delivered and from which the anchor is deployed. The anchor garage, as referenced herein, is typically a tubular element with a lumen sized, shaped, configured, and/or dimensioned to house an anchor therein so that the anchor garage may be delivered, with the anchor therein, to a target site within a patient's body. The distal end of the anchor garage may have a sharp, needle tip (e.g., configured to penetrate tissue), or may be blunt. Further in accordance with various principles of the present disclosure, an atraumatic shield is movably mounted with respect to the anchor garage to indicate and/or to control the depth of insertion of the anchor into tissue. The shield may be spring biased to extend over the distal end of the anchor garage. However, when the anchor delivery and deployment device engages with tissue at the deployment site, the atraumatic shield proximally retracts (e.g., against the spring biasing force) with respect to the distal end of the anchor garage. If the distal end of the anchor garage is sharp, then the atraumatic shield may shield the sharp end of the anchor garage prior to retracting and allowing the anchor garage to be advanced into the tissue at the deployment site for the anchor. The anchor delivery and deployment device may include a limit stop for the atraumatic shield. Once the atraumatic shield reaches the limit stop, the anchor garage is properly positioned to achieve the desired deployment for the anchor. In some instance, the limit stop serves to limit the extent to which the distal end of the anchor garage may extend beyond the atraumatic shield and into the tissue. The anchor delivery and deployment device may include any of a variety of indicators which allow the medical professional deploying the anchor to determine that tissue has been engaged by the anchor delivery and deployment device and/or that the desired depth of insertion has been achieved. For instance, the limit stop may serve as an indicator that distal end of the anchor garage is sufficiently engaged with tissue to be confident that the anchor will be properly deployed into tissue, such as achieving full deployment of the anchor at a desired depth within the tissue. Other indicators may be positioned and/or configured to provide an indication of the relative positions of the anchor garage and the atraumatic shield. Additionally or alternatively, the indicators may generate signals indicating the relative positions of the anchor garage and the atraumatic shield. In instances in which the distal end of the anchor garage is relatively blunt and not configured to pierce tissue at the target site, indication that the anchor delivery and deployment device has engaged tissue, such as to a desired extent, allows a medical professional to deploy the anchor from the anchor garage once appropriate tissue contact has been achieved, thereby enhancing deployment of the anchor into the tissue (e.g., ensuring firm securement of the anchor with tissue). As will be appreciated, delivery, deployment, and engagement of anchors may be facilitated by use of known medical visualization techniques, such as fluoroscopy, ultrasound, intra-cardiac echography, or the like. The indicators may be configured to be imaged or otherwise sensed with such techniques or other techniques known to those of ordinary skill in the art, the present disclosure not being limited in this regard.

Examples of embodiments of devices described herein provide anchoring for various devices, systems, or tools with respect to anatomical structure such as, but not limited to, the heart. One example of a procedure which may utilize devices, systems, and methods of the present disclosure is treatment of heart disease. More particularly, devices, systems, and methods described herein may be used with devices or systems for repositioning, repair, and/or replacement of one or more leaflets of a valve and/or chordae tendinea to treat heart disease. Repositioning, repair, and/or replacement of one or more leaflets of a valve and/or chordae tendinea may include one or more devices to be fixed to one or more leaflets of a heart valve and to cardiac tissue, such as papillary muscle tissue. A leaflet clip may be attached to a heart valve leaflet, and an artificial chordae tendineae extended therefrom and anchored with respect to the heart, such as to cardiac tissue such papillary muscle tissue, with an anchor.

The anchor may include a plurality of tissue engaging talons configured to pierce the tissue into which the anchor is to be anchored. In accordance with various principles of the present disclosure, the anchor is delivered in a delivery configuration in an anchor garage provided with an atraumatic shield such as described above. The delivery configuration may be a compact or compressed configuration. The anchor is moved out of the anchor garage and shifts into a deployment configuration. The deployment configuration may be an open or expanded configuration with an outer dimension greater than the outer dimension of the anchor when in a delivery configuration, and greater than the inner diameter of the anchor garage.

The anchor talons may be biased into (and held in place in) the delivery configuration for delivery within the anchor garage to the deployment site. The talons may be biased to extend into a deployed configuration, such as a curved or bowed configuration, when extended out of the anchor garage to be anchored with respect to tissue at the deployment site. For instance, the talons may be biased to extend into a deployed expanded configuration as a neutral configuration of the talons. The anchor garage may constrain or hold the anchor talons in a compact configuration to fit within the inner diameter of the anchor garage. In some embodiments, the anchor talons are extended or elongated in the compact configuration to extend along a longitudinal axis of the anchor garage. An actuator, such as a pusher rod, may be used to move the anchor out of the anchor garage for deployment in body tissue. Once the anchor is no longer within the anchor delivery and deployment device, the anchor talons may move into the open configuration, such as an expanded configuration. If the talons are spring biased into the deployment configuration, it is important to advance the distal tips of the talons into tissue so that the talons expand when in the tissue at the deployment site, rather than expanding outside the tissue. The distal ends of the anchor talons may be configured to pierce and to penetrate into the tissue and spread into a deployed configuration secured within the body tissue.

Examples of devices, systems, and methods with which embodiments of the present disclosure may be implemented include, but are not limited to, those described in U.S. Patent Application Publication US2021/0000597, titled Devices, Systems, And Methods For Adjustably Tensioning An Artificial Chordae Tendineae Between A Leaflet And A Papillary Muscle Or Heart Wall, and published on January 7, 2021; U.S. Patent Application Publication US2021/0000598, titled Devices, Systems, And Methods For Anchoring An Artificial Chordae Tendineae To A Papillary Muscle Or Heart Wall, and published on January 7, 2021; U.S. Patent Application Publication US2022/0096235, titled Devices, Systems, And Methods For Adjustably Tensioning Artificial Chordae Tendineae In A Heart, and published on March 31, 2022; U.S. Patent Application Publication US2023/0062599, titled Devices, Systems, And Methods For Anchoring An Artificial Chordae Tendineae To Cardiac Tissue, and published on March 2, 2023; and U.S. Patent Application Publication US2023/0123832, titled Devices, Systems, And Methods For Clamping A Leaflet Of A Heart Valve, and published on April 20, 2023. Examples of devices described therein may be modified to incorporate embodiments or one or more features of the present disclosure.

Examples of embodiments of devices described herein may provide anchoring for other devices, systems, or tools with respect to anatomical structure such as the heart. It will be appreciated that devices and systems described herein may be used with devices or systems disclosed in the above-referenced applications, or may be used with other devices and systems, such as those described herein.

It will be appreciated that even though the accompanying drawings illustrate a heart ventricle, and repair of a mitral valve, principles of the present disclosure are applicable to other cardiac structures, including, without limitation, the tricuspid valve. Moreover, although the present disclosure illustrates repair of leaflets and anchoring of artificial chordae tendineae to papillary muscle, principles of the present disclosure are applicable to devices other than the illustrated anchors, and to procedures other than leaflet repair (whether or not involving anchoring an artificial chordae tendineae to heart tissue) and other than cardiac procedures.

Various embodiments of devices, systems, and methods for delivering and deploying an implantable device, such as anchor, will now be described with reference to examples illustrated in the accompanying drawings. Reference in this specification to "one embodiment," "an embodiment," "some embodiments", "other embodiments", etc. indicates that one or more particular features, structures, concepts, and/or characteristics in accordance with principles of the present disclosure may be included in connection with the embodiment. However, such references do not necessarily mean that all embodiments include the particular features, structures, concepts, and/or characteristics, or that an embodiment includes all features, structures, concepts, and/or characteristics. Some embodiments may include one or more such features, structures, concepts, and/or characteristics, in various combinations thereof. It should be understood that one or more of the features, structures, concepts, and/or characteristics described with reference to one embodiment can be combined with one or more of the features, structures, concepts, and/or characteristics of any of the other embodiments provided herein. That is, any of the features, structures, concepts, and/or characteristics described herein can be mixed and matched to create hybrid embodiments, and such hybrid embodiment are within the scope of the present disclosure. Moreover, references to "one embodiment," "an embodiment," "some embodiments", "other embodiments", etc. in various places in the specification are not necessarily all referring to the same embodiment, nor are separate or alternative embodiments necessarily mutually exclusive of other embodiments. It should further be understood that various features, structures, concepts, and/or characteristics of disclosed embodiments are independent of and separate from one another, and may be used or present individually or in various combinations with one another to create alternative embodiments which are considered part of the present disclosure. Therefore, the present disclosure is not limited to only the embodiments specifically described herein, as it would be too cumbersome to describe all of the numerous possible combinations and subcombinations of features, structures, concepts, and/or characteristics, and the examples of embodiments disclosed herein are not intended as limiting the broader aspects of the present disclosure. It should be appreciated that various dimensions provided herein are examples and one of ordinary skill in the art can readily determine the standard deviations and appropriate ranges of acceptable variations therefrom which are covered by the present disclosure and any claims associated therewith. The following description is of illustrative examples of embodiments only, and is not intended as limiting the broader aspects of the present disclosure.

It will be appreciated that common features are identified by common reference elements and, for the sake of brevity and convenience, and without intent to limit, the descriptions of the common features are generally not repeated. For purposes of clarity, not all components having the same reference number are numbered. Moreover, a group of similar elements may be indicated by a number and letter, and reference may be made generally to one or such elements or such elements as a group by the number alone (without including the letters associated with each similar element). It will be appreciated that, in the following description, elements or components similar among the various illustrated embodiments with reference numbers greater than 100 are generally designated with the same reference numbers increased by a multiple of 100 and redundant description is generally omitted for the sake of brevity. Moreover, certain features in one embodiment may be used across different embodiments and are not necessarily individually labeled when appearing in different embodiments.

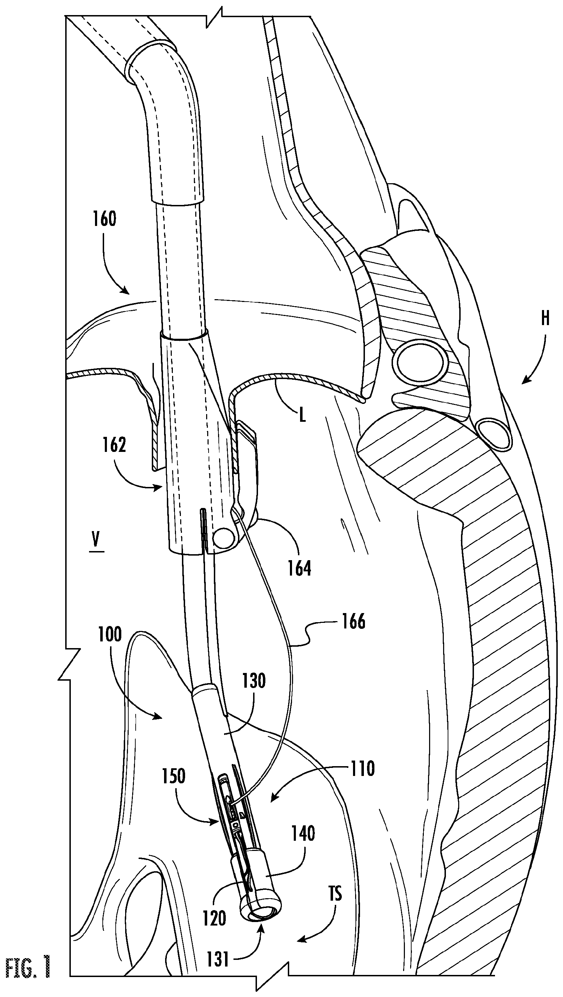

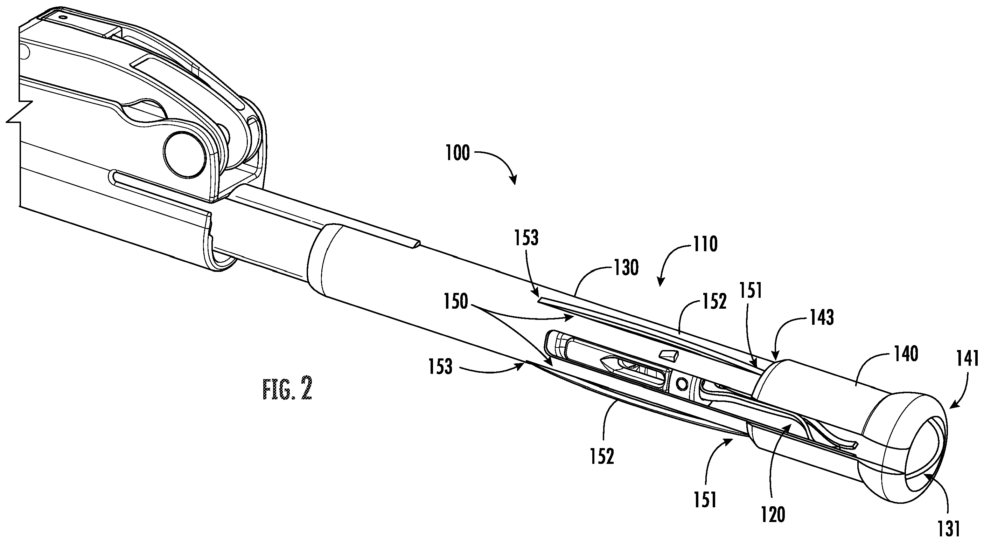

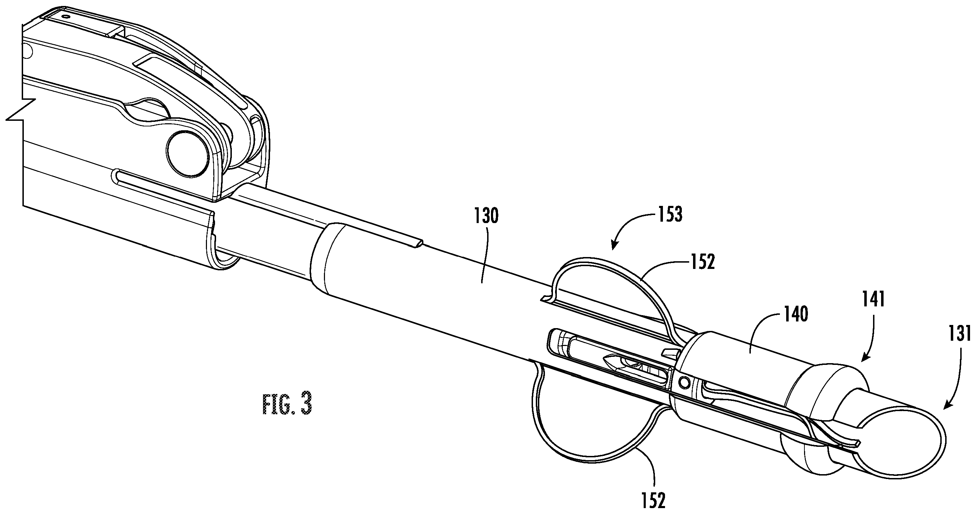

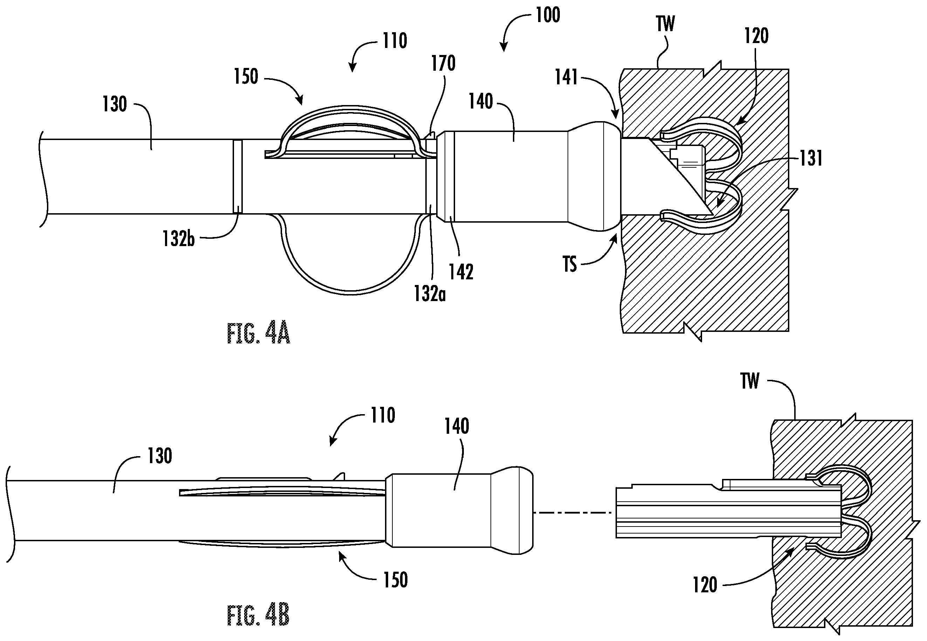

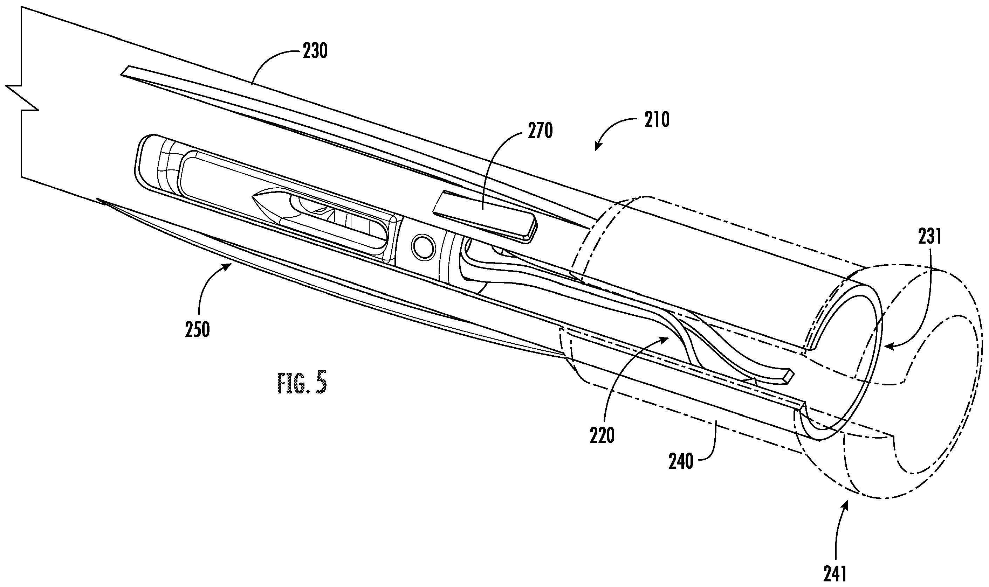

Turning now to the drawings, an anchor delivery and deployment system **100** formed in accordance with various principles of the present disclosure is illustrated in **FIG. 1** positioned to deploy an anchor into a wall of a heart. The anchor delivery and deployment system **100** includes an anchor delivery and deployment device **110** and an anchor **120.** The anchor delivery and deployment device **110** includes an anchor garage **130** configured for delivery and deployment of the anchor **120.** The anchor garage **130** may alternately be referenced herein as a delivery housing, sheath, etc., without intent to limit. The anchor **120** is delivered within the anchor garage **130** in a generally unexpanded delivery configuration, as may be appreciated with reference to **FIG. 2** and **FIG. 3****.** In the example of an embodiment illustrated in **FIG. 2****,** the anchor garage **130** has a sharp distal tip **131** configured to pierce tissue at the target site **TS** to facilitate deployment of the anchor **120,** as discussed in further detail below with reference to **FIG. 4A** and **FIG. 4B****.** Alternatively, the distal tip of the anchor garage may be blunt, as illustrated in **FIG. 5****,** as discussed in further detail below.

In accordance with various principles of the present disclosure, an atraumatic shield **140** is movably positioned with respect to the distal tip **131** of the anchor garage **130.** The atraumatic shield **140** is configured to move proximally with respect to the anchor garage **130,** such as upon contact with tissue at the target site **TS.** In some embodiments, the atraumatic shield **140** covers the distal tip **131** of the anchor garage **130** during delivery, and is proximally retractable to uncover the distal tip **131** of the anchor garage **130,** such as when the anchor **120** is to be deployed from the anchor garage **130.** Movement of the atraumatic shield **140** may provide confirmation of contact of the anchor delivery and deployment device **110** with tissue and/or may ensure engagement of the anchor **120** with tissue to ensure full and complete deployment of the anchor **120** (e.g., confirming / ensuring that all talons thereof are deployed in tissue) and/or may ensure the desired depth of penetration and deployment of the anchor **120,** and may provide other various benefits, such as described in further detail below.

The atraumatic shield **140** may be biased distally toward the distal end **131** of the anchor garage **130** by a biasing element **150.** The biasing element **150** holds the atraumatic shield **140** in a distal position adjacent (e.g., covering) the distal end **131** of the anchor garage **130,** such as illustrated in **FIG. 1** and **FIG. 2****.** The biasing element **150** may move (e.g., contract or bend or bow or otherwise change configuration) to allow proximal retraction of the atraumatic shield **140** with respect to the anchor garage **130,** such as illustrated in **FIG. 3****.** For instance, application of a force to the distal end **141** of the atraumatic shield **140** and/or encountering of the distal end **141** of the atraumatic shield **140** with another object, such as a tissue wall (e.g., at the target site **TS**) may cause the biasing element **150** to allow retraction of the atraumatic shield **140.** The biasing element **150** may be configured to return the atraumatic shield **140** to a distal position upon removal of a force against or obstruction with respect to the atraumatic shield **140.** For instance, in some instances, it may be desirable to retract the anchor delivery and deployment device **110** into a tubular element **160** in which the anchor delivery and deployment device **110** may be delivered transluminally within a patient's body to a target site **TS** (such as illustrated in **FIG. 1**). The tubular element **160** may be a delivery shaft, or another device such as a leaflet clip delivery device **162** as illustrated in **FIG. 1** (such device delivering and deploying a leaflet clip **164** on a heart valve leaflet with an artificial chordae tendineae **166** extending therefrom to the anchor **120** within the anchor garage **130**). In such instances, it may be desirable for the biasing element **150** to return to a configuration allowing the anchor delivery and deployment device **110** to be retracted into a tubular element such as one or more tubular elements in which the anchor delivery and deployment device **110** had been delivered to the target site **TS.**

In the example of an embodiment illustrated in **FIG. 2****,** the biasing element **150** includes one or more resilient beams **152.** The beams **152** hold the atraumatic shield **140** in a distal position adjacent (e.g., covering) the distal end **131** of the anchor garage **130,** such as illustrated in **FIG. 1** and **FIG. 2****.** The distal ends **151** of the beams engage or contact the atraumatic shield **140** and the proximal ends **153** of the beams **152** engage or contact the anchor garage **130.** For instance, the distal ends **151** and/or the proximal ends **153** may be coupled to and/or fixed with respect to the atraumatic shield **140** and the anchor garage **130** respectively. It will be appreciated that terms such as engage, contact, couple, fix, etc., and other grammatical forms thereof, may be used interchangeably herein without intent to limit unless explicitly indicated. In some embodiments, the beams **152** are integrally formed with the anchor garage **130,** such as by being cut (e.g., laser cut) from a wall of the anchor garage **130.** The distal ends **151** of the beams **152** may be detached from the anchor garage **130** and attached (in any desired manner known to those of ordinary skill in the art, such as welding, soldering, brazing, or mechanical coupling such as an interference fit) to the atraumatic shield **140.** In some embodiments, the beams **152** are formed separately from the anchor garage **130** and optionally fitted over the wall of the anchor garage **130** or within cutouts within the wall of the anchor garage **130.** The distal ends **151** of the beams may be attached to the atraumatic shield **140** in a manner as described above. The proximal ends **153** may be attached to the anchor garage **130** in a manner similar to the manner in which the proximal ends **153** are attached to the atraumatic shield **140.** Alternatively, the ends **151, 153** may be held with respect to the anchor garage **130** and/or the atraumatic shield **140** with a friction or interference fit or other mechanical coupling such as known to those of ordinary skill in the art.

Resilient beams **152** of an example of an embodiment of a biasing element **150** as illustrated in **FIG. 2** and **FIG. 3** may flex, bend, bow, etc., to allow proximal retraction of the atraumatic shield **140** with respect to the anchor garage **130,** such as illustrated in **FIG. 3****.** For instance, application of a force to the distal end **141** of the atraumatic shield **140** and/or encountering of the distal end **141** of the atraumatic shield **140** with another object, such as a tissue wall (e.g., at the target site **TS**), may cause the beams **152** to flex, bend, bow, etc., to allow retraction of the atraumatic shield **140.** It will be appreciated that terms such as flex, bend, bow, contract, deform, deflect, etc., including other grammatical forms thereof, may be used interchangeably herein without intent to limit. The beams **152** may be formed of a shape memory material (such as, without limitation, a titanium alloy such as nitinol, or stainless steel) to bias the beams **152** in a selected configuration. For instance, the shape memory of the beams **152** may return the beams **152** to a neutral, rest position, such as a generally elongated position as illustrated in **FIG. 1****,** upon removal of a force against or obstruction with respect to the atraumatic shield **140.** The beams **152** may be configured to return the atraumatic shield **140** to a distal position upon removal of a force against or obstruction with respect to the atraumatic shield **140.** The shape memory of the beams **152** may hold the atraumatic shield **140** in a distal position, either the same as the initial delivery position (before being proximally retracted) or close to such position. In instances in which the anchor delivery and deployment device **110** is retracted into a tubular element (such as a shaft **160** in which the anchor delivery and deployment device **110** has been delivered) once the anchor **120** has been deployed, the biasing element **150** may return the beams **152** to (or close to) an initial delivery position before deployment of the anchor **120** to fit back into the tubular delivery element. It will be appreciated that the flexibility of the beams **152** may be tuned or otherwise adjusted to suit the particular use, environment, etc., of the biasing element **150,** such as to adjust or to achieve the desired degree of retraction of the atraumatic shield **140.**

In accordance with various principles of the present disclosure, and as noted above, the distal end **141** of the atraumatic shield **140** may be blunt or curved or rounded otherwise configured to present an atraumatic surface for contacting tissue in an atraumatic matter. The blunt distal end **141** of the atraumatic shield **140** may be sized, shaped, configured, and dimensioned to facilitate pushing of the atraumatic shield **140** against cardiac tissue without pushing the distal end **141** of the atraumatic shield **140** into the cardiac tissue as well. For instance, the distal end **141** of the atraumatic shield **140** may be radiused or curved inwardly (presenting a convex curved outer surface) or otherwise formed to be sufficiently blunt as to not injure tissue against which the atraumatic shield **140** is pushed. For example, and without limitation, edges of the distal end **141** of the atraumatic shield **140** may be curved with a radius of approximately 0.0275 in (0.6985 mm). Pressing of the distal end **141** of the atraumatic shield **140** against tissue may cause the atraumatic shield **140** to proximally retract (such as if the distal end **131** of the anchor garage **130** is proximal to the distal end **141** of the atraumatic shield **140**). Such retraction may provide an indication to the medical professional of one or more conditions of interest to the medical professional. For instance, proximal retraction of the atraumatic shield **140** may be used to indicate the position of the anchor delivery and deployment device **110** and/or to control and/or limit advancement of the anchor garage **130** and/or the anchor **120** into tissue as will be explained with reference to the example of an embodiment illustrated in **FIG. 4A** and **FIG. 4B****,** and the example of an embodiment illustrated in **FIG. 5****.**

In the example of an embodiment illustrated in **FIG. 4A** and **FIG. 4B****,** an anchor delivery and deployment device **110** such as illustrated in **FIG. 2** and **FIG. 3****,** is advanced into contact with a tissue wall **TW** at a target site **TS.** As the anchor delivery and deployment device **110** is advanced distally, the blunt distal end **141** of the atraumatic shield **140** does not advance distally, and, instead, is proximally retracted with respect to the anchor garage **130** and against the biasing force of the biasing element **150.** The anchor delivery and deployment device **110** is provided with one or more indicators configured to provide information about the atraumatic shield **140** and/or the anchor garage **130** and/or the relative positions thereof to the medical professional operating the anchor delivery and deployment system **100.** Such information is useful in determining that the anchor delivery and deployment device **110** has contacted tissue at the target site **TS** so that the anchor **120** may be securely deployed into the tissue at the target site **TS.**

In some embodiments, at least a portion of the atraumatic shield **140** and/or the anchor garage **130** is formed of a material which is imageable with any of a variety of imaging systems, such as fluoroscopy and/or ultrasound and/or echocardiography, so that the medical professional operating the anchor delivery and deployment system **100** may view when the atraumatic shield **140** is proximally retracted. Such material may serve as an indicator. In some embodiments, the indicator is a limited extent of the atraumatic shield **140** and/or the anchor garage **130** configured at least to enable the medical professional to view movement of the atraumatic shield **140,** and optionally also the relative positions of the atraumatic shield **140** and the anchor garage **130.** In some embodiments, the atraumatic shield **140** includes an indicator **142** in the form of one or more visualization markers, such as radiopaque markers, bands, or radiopaque filler materials, and the anchor garage **130** includes a corresponding, typically similarly formed indicator **132a, 132b.** In some embodiments, visualization markers may be provided on just the anchor garage **130,** so that as the atraumatic shield **140** moves proximally, the atraumatic shield **140** covers or masks the visualization markers on the anchor garage **130.** The medical professional may then be able to determine the position of the atraumatic shield **140** relative to the anchor garage **130** such as if all visualization markers are masked or a certain number of the visualization markers (e.g., 2/3 of the markers) are masked. The position of an indicator **142** on the atraumatic shield **140** relative to an indicator **132a, 132b** on the anchor garage **130** may be viewed with any of a variety of appropriate imaging systems to provide information to a medical professional with regard to the position, configuration, status, etc., of the anchor delivery and deployment device **110,** such as with respect to the target site **TS.** The relative positions of the anchor garage **130** and the atraumatic shield **140** may serve to indicate that the distal end **131** of the anchor garage **130** is engaged with tissue in a manner ensuring the desired deployment therefrom of the anchor **120** housed therein. The relative positions of the anchor garage **130** and the atraumatic shield **140** may indicate there is adequate force / depth of insertion of the anchor **120** even for a blunt system (e.g., if the distal end **131** of the anchor garage **130** is blunt, as discussed in further detail below). For instance, adequate force may be achieved when the entirety of the distal end **131** of the anchor garage **130** is in contact with the tissue wall. Optionally, an indicator may be provided on the anchor garage **130** and/or the atraumatic shield **140** which emits a signal (audio, visual, etc.) when the atraumatic shield **140** has been retracted distally by a selected distance. Such indicator may be actuated upon meeting of elements of the atraumatic shield **140** and the anchor garage **130** in a manner known to those of ordinary skill in the art. For example, electrical sensors may be provided on the atraumatic shield **140** and the anchor garage **130** to generate a signal (a sound, or a visual signal such as illuminating an LED) when the atraumatic shield **140** is fully retracted.

Information from an indicator with regard to relative positions of an atraumatic shield and an anchor garage extending therethrough may be useful to identify for a medical practitioner when an anchor delivery and deployment device has contacted tissue so that a device may be deployed (e.g., may be implanted in such tissue). For instance, once an anchor delivery and deployment device **110** such as illustrated in **FIG. 4A** and **FIG. 4B** has contacted a tissue wall **TW,** the anchor **120** of the anchor delivery and deployment system **100** may be advanced distally into the tissue wall **TW,** as illustrated in **FIG. 4A****.** In embodiments in which the anchor garage **130** has a sharp distal end **131,** the distal end **131** of the anchor garage **130** may also be advanced distally into the target site **TS,** such as to facilitate deployment of the anchor **120,** such as illustrated in **FIG. 4A****,** and discussed in greater detail below. However, in the example of an embodiment of an anchor delivery and deployment device **210** illustrated in **FIG. 5****,** the distal end **231** of the anchor garage **230** is generally blunt and not configured to penetrate tissue. In a neutral, delivery configuration, such as illustrated in **FIG. 5****,** the distal end **241** of the atraumatic shield **240** (shown in phantom so that the relative position of the anchor garage **230** therein may be viewed) extends distal to the distal end **231** of the anchor garage **230.** As such, upon contact of the atraumatic shield **240** with a tissue wall (e.g., a tissue wall **TW as** illustrated in **FIG. 4A** and **FIG. 4B**), and continued distal advancement of the anchor delivery and deployment device **210,** the atraumatic shield **240** may be retracted or pushed proximally with respect to the anchor garage **230** with respect to which (e.g., over which) the atraumatic shield **240** extends. The proximal movement of the atraumatic shield **240** relative to the anchor garage **230** may continue until the blunt distal end **231** of the anchor garage **230** (initially proximal to the distal end **241** of the atraumatic shield **240** and thus not contacting the tissue wall) contacts the tissue wall as well, at which point the effect of such contact on the atraumatic shield **240** is minimized if not removed. When such position is achieved, the medical practitioner may receive a tactile indication (e.g., resistance to further advancement of the anchor delivery and deployment device **210**). Additionally or alternatively, any of a variety of indicators, such as described above with respect to the anchor delivery and deployment device **110** illustrated in **FIG. 2****,** **FIG. 3****,** **FIG. 4A,** and **FIG. 4B****,** may be used. For instance, an indicator which may be imaged by any of a variety of imaging systems may be provided on the atraumatic shield **240** and/or the anchor garage **230** so that relative movement therebetween may be observed, including whether the desired relative movement has been achieved (e.g., indicating sufficient contact with tissue for proper, complete, full, etc., deployment of the anchor **220**).

In some embodiments, a limit stop **170** may be provided to limit proximal retraction of the atraumatic shield **140.** The limit stop **170** may be yet another indicator that the anchor garage **130** has been fully extended and/or is in full contact with tissue so that the anchor **120** may be deployed to the desired depth within tissue. As noted above, in some embodiments, such as illustrated in **FIG. 4A** and **FIG. 4B****,** an anchor garage **130** of an anchor delivery and deployment device **110** may have a sharp distal end **131** which may be sharp enough to penetrate a tissue wall **TW** once the atraumatic shield **140** has been sufficiently proximally retracted to expose a sufficient extent of such sharp distal end **131.** In such embodiments, in addition to being an indicator which allows a medical practitioner to determine or to observe relative positions of the atraumatic shield **140** and the anchor garage **130** to determine proper positioning for the anchor garage **130** for deployment of the anchor **120,** the limit stop **170** may limit the depth to which the sharp distal end **131** of the anchor garage **130** can penetrate the tissue wall **TW.** The limit stop **170** may be a protrusion (e.g., detent or weld) such as illustrated in **FIG. 4A****.** Additionally or alternatively, a limit stop may be formed from part of the anchor garage, such as from a punched-out tab limit stop **270** such as illustrated in **FIG. 5****.** As may be appreciated, a limit stop **170, 270** may provide various benefits regardless of whether the distal end of the anchor garage is sharp or blunt.

Once the anchor **120, 220** has been deployed from the anchor garage **130, 230,** the biasing element **150, 250** biases the atraumatic shield **140, 240** distally, such as to cover the distal end **131, 231**of the anchor garage **130, 230** and/or to facilitate withdrawal of the anchor delivery and deployment device **110, 220** into a delivery shaft (e.g., the delivery shaft in which the anchor delivery and deployment device **110, 220** has been delivered), such as for withdrawal from the patient. For instance, as illustrated in **FIG. 4B****,** once the anchor **120** has been deployed in the tissue wall **TW,** the biasing element **150** may return the atraumatic shield **140** to a position covering the sharp distal end **131** of the anchor garage **130,** such as to protect tissue from inadvertent contact with the sharp distal end **131.**

It will be appreciated that other features of the anchor delivery and deployment system **100** with the anchor delivery and deployment device **210** illustrated in **FIG. 5** may be substantially similar to those of **FIGS. 1-3****, 4A,** and **4B,** reference being made to those figures and the associated descriptions of elements and features illustrated therein for the sake of brevity. For instance, various features of the anchor delivery and deployment devices **110, 220** described above can be arranged and operate in substantially the same or similar manners. Accordingly, for the sake of brevity and convenience, and without intent to limit, common elements with common functions are indicated with the same reference characters differing in value by 100, reference being made to the above descriptions of similar elements and operations.

It will be appreciated that all apparatuses and methods discussed herein are examples of apparatuses and/or methods implemented in accordance with one or more principles of this disclosure. These examples are not the only way to implement these principles but are merely examples, not intended as limiting the broader aspects of the present disclosure. Thus, references to elements or structures or features in the drawings must be appreciated as references to examples of embodiments of the disclosure, and should not be understood as limiting the disclosure to the specific elements, structures, or features illustrated. Other examples of manners of implementing the disclosed principles will occur to a person of ordinary skill in the art upon reading this disclosure.

It will further be appreciated that any or all of the aspects of the above-described delivery and deployment may be performed using available appropriate imaging techniques such as known or heretofore known to those of ordinary skill in the art.

As noted above, although embodiments of the present disclosure may be described with specific reference to medical devices and systems and procedures for deploying (e.g., anchoring) a device with respect to tissue of a cardiovascular system (e.g., heart tissue), it should be appreciated that such medical devices and methods may be used to treat tissues of the gastrointestinal system, the abdominal cavity, etc. It is to be understood by one of ordinary skill in the art that the present discussion is a description of illustrative examples of embodiments only, and is not intended as limiting the broader aspects of the present disclosure.

The foregoing discussion has broad application and has been presented for purposes of illustration and description and is not intended to limit the disclosure to the form or forms disclosed herein. It will be understood that various additions, modifications, and substitutions may be made to embodiments disclosed herein without departing from the concept and and scope of the present disclosure. In particular, it will be clear to those skilled in the art that principles of the present disclosure may be embodied in other forms, structures, arrangements, proportions, and with other elements, materials, and components, without departing from the concept , or scope, or characteristics thereof. For example, various features of the disclosure are grouped together in one or more aspects, embodiments, or configurations for the purpose of streamlining the disclosure. However, it should be understood that various features of the certain aspects, embodiments, or configurations of the disclosure may be combined in alternate aspects, embodiments, or configurations. While the disclosure is presented in terms of embodiments, it should be appreciated that the various separate features of the present subject matter need not all be present in order to achieve at least some of the desired characteristics and / or benefits of the present subject matter or such individual features. One skilled in the art will appreciate that the disclosure may be used with many modifications or modifications of structure, arrangement, proportions, materials, components, and otherwise, used in the practice of the disclosure, which are particularly adapted to specific environments and operative requirements without departing from the principles or scope of the present disclosure. For example. elements shown as integrally formed may be constructed of multiple parts or elements shown as multiple parts may be integrally formed, the operation of elements may be reversed or otherwise varied, the size or dimensions of the elements may be varied. Similarly, while operations or actions or procedures are described in a particular order, this should not be understood as requiring such particular order, or that all operations or actions or procedures are to be performed, to achieve desirable results. The presently disclosed embodiments are therefore to be considered in all respects as illustrative and not restrictive, the scope of the claimed subject matter being indicated by the appended claims, and not limited to the foregoing description or particular embodiments or arrangements described or illustrated herein.

In the foregoing description and the following claims, the following will be appreciated. The phrases "at least one", "one or more", and "and/or", as used herein, are open-ended expressions that are both conjunctive and disjunctive in operation. The terms "a", "an", "the", "first", "second", etc., do not preclude a plurality. For example, the term "a" or "an" entity, as used herein, refers to one or more of that entity. As such, the terms "a" (or "an"), "one or more" and "at least one" can be used interchangeably herein. As used in this specification and the appended claims, the term "or" is generally employed in its sense including "and/or" unless the content clearly dictates otherwise. As used herein, the conjunction "and" includes each of the structures, components, features, or the like, which are so conjoined, unless the context clearly indicates otherwise, and the conjunction "or" includes one or the others of the structures, components, features, or the like, which are so conjoined, singly and in any combination and number, unless the context clearly indicates otherwise. All directional references (e.g., proximal, distal, upper, lower, upward, downward, left, right, lateral, longitudinal, front, back, top, bottom, above, below, vertical, horizontal, radial, axial, clockwise, counterclockwise, and/or the like) are only used for identification purposes to aid the reader's understanding of the present disclosure, and/or serve to distinguish regions of the associated elements from one another, and do not limit the associated element, particularly as to the position, orientation, or use of this disclosure. Connection references (e.g., attached, coupled, connected, engaged, and joined) are to be construed broadly and may include intermediate members between a collection of elements and relative movement between elements unless otherwise indicated. As such, connection references do not necessarily infer that two elements are directly connected and in fixed relation to each other. Identification references (e.g., primary, secondary, first, second, third, fourth, etc.) are not intended to connote importance or priority, but are used to distinguish one feature from another.

## Claims

1. An anchor delivery and deployment device (110) comprising:
an anchor garage (130, 230) configured to deliver an anchor (120, 220) therein;
an atraumatic shield (140, 240) movable with respect to said anchor garage (130, 230);
and
a biasing element (150, 250) **characterised in that** the biasing element (150, 250) comprises a plurality of elongated beams (152) proximal to said atraumatic shield (140, 240) and biased into an elongated configuration along said anchor garage (130, 230) to bias said atraumatic shield (140, 240) toward the distal end (131, 141, 151, 231, 241) of said anchor garage (130, 230).

2. The anchor delivery and deployment device (110) of claim 1, further comprising an indicator (132a, 132b, 142) of the relative positions of said anchor garage (130, 230) and said atraumatic shield (140, 240).

3. The anchor delivery and deployment device (110) of claim 2, wherein said indicator (132a, 132b, 142) is a visual indicator.

4. The anchor delivery and deployment device (110) of any one of claims 2-3, wherein said indicator (132a, 132b, 142) includes at least one radiopaque marker.

5. The anchor delivery and deployment device (110) of any one of claims 2-4, wherein said indicator (132a, 132b, 142) generates a signal indicating the relative positions of said anchor garage (130, 230) and said atraumatic shield (140, 240).

6. The anchor delivery and deployment device (110) of any one of claims 2-5, wherein said indicator (132a, 132b, 142) indicates that said anchor delivery and deployment device has contacted tissue.

7. The anchor delivery and deployment device (110) of any one of claims 1-6, further comprising a limit stop (170, 270) positioned to limit relative movement between said atraumatic shield (140, 240) and said anchor garage (130, 230).

8. The anchor delivery and deployment device (110) of claim 7, wherein said limit stop (170, 270) is positioned and configured to limit proximal retraction of said atraumatic shield (140, 240) with respect to said anchor garage (130, 230).

9. The anchor delivery and deployment device (110) of any one of claims 1-8, wherein the distal end (131, 141, 151, 231, 241) of said anchor garage (130, 230) is sharp to penetrate tissue, and said biasing element (150, 250) biases said atraumatic shield (140, 240) over the distal end (131, 141, 151, 231, 241) of said anchor garage (130, 230).

10. The anchor delivery and deployment device (110) of claim 1, wherein said beams (152) are formed from the wall of said anchor garage (130, 230).

11. An anchor delivery and deployment system (100) comprising the anchor delivery and deployment device (110) of claim 1 and an anchor (120, 220).

12. The anchor delivery and deployment system (100) of claim 11, further comprising a tubular delivery element configured to deliver said anchor, said anchor garage (130, 230), and said atraumatic shield (140, 240) transluminally through a patient's body to a target site.

13. The anchor delivery and deployment system (100) of any one of claims 11-12, further comprising an indicator (132a, 132b, 142) configured to indicate the relative positions of said anchor garage (130, 230) and said atraumatic shield (140, 240).

14. The anchor delivery and deployment system (100) of any one of claims 11-13, further comprising a limit stop (170, 270) positioned to limit relative movement of said atraumatic shield (140, 240) with respect to said anchor garage (130, 230).

15. The anchor delivery and deployment device (110) of any one of claims 1-10, wherein:
the anchor (120, 220) includes a plurality of tissue-engaging talons biased to extend into a deployed configuration when extended out of the anchor garage (130, 230), the talons being configured to pierce and secure the anchor (120, 220) within the tissue.

## Patentansprüche

1. Ankerzuführ- und -Einsetzvorrichtung (110) umfassend:
eine Ankergarage (130, 230), ausgelegt zum Abgeben eines Ankers (120, 220) darin;
eine atraumatische Abschirmung (140, 240), die bezogen auf die Ankergarage (130, 230) beweglich ist; und
ein Vorspannelement (150, 250), **dadurch gekennzeichnet, dass** das Vorspannelement (150, 250) mehrere langgestreckte Stäbe (152) proximal zu der atraumatischen Abschirmung (140, 240) umfasst und in eine langgestreckte Konfiguration entlang der Ankergarage (130, 230) vorgespannt ist, um die atraumatische Abschirmung (140, 240) in Richtung zu dem distalen Ende (131, 141, 151, 231, 241) der Ankergarage (130, 230) vorzuspannen.

2. Ankerzuführ- und -Einsetzvorrichtung (110) nach Anspruch 1, ferner umfassend einen Indikator (132a, 132b, 142) der relativen Positionen der Ankergarage (130, 230) und der atraumatischen Abschirmung (140, 240).

3. Ankerzuführ- und -Einsetzvorrichtung (110) nach Anspruch 2, wobei der Indikator (132a, 132b, 142) ein visueller Indikator ist.

4. Ankerzuführ- und -Einsetzvorrichtung (110) nach einem der Ansprüche 2-3, wobei der Indikator (132a, 132b, 142) wenigstens einen röntgendichten Marker umfasst.

5. Ankerzuführ- und -Einsetzvorrichtung (110) nach einem der Ansprüche 2-4, wobei der Indikator (132a, 132b, 142) ein Signal erzeugt, das die relativen Positionen der Ankergarage (130, 230) und der atraumatischen Abschirmung (140, 240) anzeigt.

6. Ankerzuführ- und -Einsetzvorrichtung (110) nach einem der Ansprüche 2-5, wobei der Indikator (132a, 132b, 142) anzeigt, dass die Ankerzuführ- und -Einsetzvorrichtung mit Gewebe Kontakt gebildet hat.

7. Ankerzuführ- und -Einsetzvorrichtung (110) nach einem der Ansprüche 1-6, ferner umfassend einen Begrenzungsanschlag (170, 270), der angeordnet ist, um Relativbewegung zwischen der atraumatischen Abschirmung (140, 240) und der Ankergarage (130, 230) zu begrenzen.

8. Ankerzuführ- und -Einsetzvorrichtung (110) nach Anspruch 7, wobei der Begrenzungsanschlag (170, 270) angeordnet und gestaltet ist, um proximales Zurückziehen der atraumatischen Abschirmung (140, 240) bezogen auf die Ankergarage (130, 230) zu begrenzen.

9. Ankerzuführ- und -Einsetzvorrichtung (110) nach einem der Ansprüche 1-8, wobei das distale Ende (131, 141, 151, 231, 241) der Ankergarage (130, 230) scharf ist, um Gewebe zu durchdringen, und das Vorspannelement (150, 250) die atraumatische Abschirmung (140, 240) über das distale Ende (131, 141, 151, 231, 241) der Ankergarage (130, 230) vorspannt.

10. Ankerzuführ- und -Einsetzvorrichtung (110) nach Anspruch 1, wobei die Stäbe (152) aus der Wand der Ankergarage (130, 230) gebildet sind.

11. Ankerzuführ- und -Einsetzsystem (100) umfassend die Ankerzuführ- und - Einsetzvorrichtung (110) nach Anspruch 1 und einen Anker (120, 220).

12. Ankerzuführ- und -Einsetzsystem (100) nach Anspruch 11, ferner umfassend ein röhrenförmiges Zuführelement, das dazu ausgelegt ist, den Anker, die Ankergarage (130, 230) und die atraumatische Abschirmung (140, 240) transluminal durch den Körper eines Patienten zu einer Zielstelle zuzuführen.

13. Ankerzuführ- und -Einsetzsystem (100) nach einem der Ansprüche 11-12, ferner umfassend einen Indikator (132a, 132b, 142), der dazu ausgelegt ist, die relativen Positionen der Ankergarage (130, 230) und der atraumatischen Abschirmung (140, 240) anzuzeigen.

14. Ankerzuführ- und -Einsetzsystem (100) nach einem der Ansprüche 11-13, ferner umfassend einen Begrenzungsanschlag (170, 270), der angeordnet ist, um Relativbewegung der atraumatischen Abschirmung (140, 240) bezogen auf die Ankergarage (130, 230) zu begrenzen.

15. Ankerzuführ- und -Einsetzvorrichtung (110) nach einem der Ansprüche 1-10, wobei:
der Anker (120, 220) mehrere Gewebeeingriffskrallen aufweist, die vorgespannt sind, um in eine eingesetzte Konfiguration vorzustehen, wenn sie aus der Ankergarage (130, 230) ausgefahren werden, wobei die Krallen dazu ausgelegt sind, das Gewebe zu durchstechen und den Anker (120, 220) darin zu sichern.

## Revendications

1. Dispositif de pose et de déploiement de dispositif d'ancrage (110) comprenant :
un logement de dispositif d'ancrage (130, 230) configuré pour y poser un dispositif d'ancrage (120, 220) ;
une protection atraumatique (140, 240) mobile par rapport audit logement de dispositif d'ancrage (130, 230) ; et
un élément de sollicitation (150, 250) **caractérisé en ce que** l'élément de sollicitation (150, 250) comprend une pluralité de lames allongées (152) proximales à ladite protection atraumatique (140, 240) et sollicitées dans une configuration allongée le long dudit logement de dispositif d'ancrage (130, 230) pour solliciter ladite protection atraumatique (140, 240) vers l'extrémité distale (131, 141, 151, 231, 241) dudit logement de dispositif d'ancrage (130, 230).

2. Dispositif de pose et de déploiement de dispositif d'ancrage (110) selon la revendication 1, comprenant en outre un indicateur (132a, 132b, 142) des positions relatives dudit logement de dispositif d'ancrage (130, 230) et de ladite protection atraumatique (140, 240).

3. Dispositif de pose et de déploiement de dispositif d'ancrage (110) selon la revendication 2, ledit indicateur (132a, 132b, 142) étant un indicateur visuel.

4. Dispositif de pose et de déploiement de dispositif d'ancrage (110) selon l'une quelconque des revendications 2 et 3, ledit indicateur (132a, 132b, 142) comprenant au moins un marqueur radio-opaque.

5. Dispositif de pose et de déploiement de dispositif d'ancrage (110) selon l'une quelconque des revendications 2 à 4, ledit indicateur (132a, 132b, 142) générant un signal indiquant les positions relatives dudit logement de dispositif d'ancrage (130, 230) et de ladite protection atraumatique (140, 240).

6. Dispositif de pose et de déploiement de dispositif d'ancrage (110) selon l'une quelconque des revendications 2 à 5, ledit indicateur (132a, 132b, 142) indiquant que ledit Dispositif de pose et de déploiement de dispositif d'ancrage a contacté un tissu.

7. Dispositif de pose et de déploiement de dispositif d'ancrage (110) selon l'une quelconque des revendications 1 à 6, comprenant en outre une butée limite (170, 270) positionnée pour limiter le mouvement relatif entre ladite protection atraumatique (140, 240) et ledit logement de dispositif d'ancrage (130, 230).

8. Dispositif de pose et de déploiement de dispositif d'ancrage (110) selon la revendication 7, ladite butée limite (170, 270) étant positionnée et configurée pour limiter la rétraction proximale de ladite protection atraumatique (140, 240) par rapport audit logement de dispositif d'ancrage (130, 230).

9. Dispositif de pose et de déploiement de dispositif d'ancrage (110) selon l'une quelconque des revendications 1 à 8, l'extrémité distale (131, 141, 151, 231, 241) dudit logement de dispositif d'ancrages (130, 230) étant pointue pour pénétrer dans le tissu, et ledit élément de sollicitation (150, 250) sollicitant ladite protection atraumatique (140, 240) sur l'extrémité distale (131, 141, 151, 231, 241) dudit logement de dispositif d'ancrages (130, 230).

10. Dispositif de pose et de déploiement de dispositif d'ancrage (110) selon la revendication 1, lesdites lames (152) étant formées à partir de la paroi dudit logement de dispositif d'ancrage (130, 230).

11. Système de pose et de déploiement de dispositif d'ancrage (100) comprenant le dispositif de pose et de déploiement de dispositif d'ancrage (110) selon la revendication 1 et un dispositif d'ancrage (120, 220).

12. Système de pose et de déploiement de dispositif d'ancrage (100) selon la revendication 11, comprenant en outre un élément de pose tubulaire configuré pour poser ledit dispositif d'ancrage, ledit logement de dispositif d'ancrage (130, 230) et ladite protection atraumatique (140, 240) de manière transluminale à travers le corps d'un patient jusqu'à un site cible.

13. Système de pose et de déploiement de dispositif d'ancrage (100) selon l'une quelconque des revendications 11 à 12, comprenant en outre un indicateur (132a, 132b, 142) configuré pour indiquer les positions relatives dudit logement de dispositif d'ancrage (130, 230) et de ladite protection atraumatique (140, 240).

14. Système de pose et de déploiement de dispositif d'ancrage (100) selon l'une quelconque des revendications 11 à 13, comprenant en outre une butée limite (170, 270) positionnée pour limiter le mouvement relatif de ladite protection atraumatique (140, 240) par rapport audit logement de dispositif d'ancrage (130, 230).

15. Dispositif de pose et de déploiement de dispositif d'ancrage (110) selon l'une quelconque des revendications 1 à 10,
le dispositif d'ancrage (120, 220) comprenant une pluralité de pattes d'ancrage s'engageant dans le tissu sollicitées pour s'étendre dans une configuration déployée lorsqu'elles sont étendues hors du logement de dispositif d'ancrage (130, 230), les pattes d'ancrage étant configurées pour percer et fixer le dispositif d'ancrage (120, 220) à l'intérieur du tissu.
